# EUROPEAN PATENT SPECIFICATION

(11) **EP 1 827 318 B1**
(45) Date of publication and mention of the grant of the patent: **21.12.2011**
(21) Application number: 05823121.8
(22) Date of filing: 04.11.2005
(51) Int. Cl.: A61F 2/40

(54) **GLENOID PROSTHESIS**
GLENOID-PROTHESE
PROTHESE GLENOIDE

(30) Priority: 05.11.2004 DE 102004053606
(43) Date of publication of application: 05.09.2007
(73) Proprietor: Smith & Nephew Orthopaedics AG, 6343 Rotkreuz (CH)
(72) Inventor: BAILIE, David,S., Scottsdale, AZ 85255 (US); RINER, Marc, A., 5628 Aristau (CH)
(74) Representative: Popp, Eugen
(86) International application number: PCT/US2005/040180
(87) International publication number: WO 2006/052833

(56) References cited:
- EP-A- 1 413 266
- FR-A- 2 704 747
- US-A- 4 964 865
- US-A- 5 782 924
- US-A1- 2004 122 520

## Description

### BACKGROUND OF THE INVENTION

### Field of the Invention

The invention relates to a glenoid prosthesis, and in particular relates to a prosthetic joint replacement for a shoulder joint, with a bearing shell the medial side of which, where appropriate, comprises anchoring elements and the lateral side of which defines a concave bearing surface.

### Description of the Related Art

Conventional shoulder joint replacement includes the replacement of either only the joint surface on the humerus side, i.e. the head of the humerus (hemi-arthroplasty), or the replacement of both joint surfaces, i.e. the head of the humerus and the glenoid fossa (total arthroplasty). Both monoblock and modular endoprostheses can be employed in shoulder joint replacement procedures.

The artificial joint replacement is usually made of metal. In particular, the artificial joint can be made of a cobalt-chromium or titanium alloy, or of pure titanium. However, ceramics and polyethylene (PE) can also be used. The implants on the humerus side are either cemented into place or installed without cement. The same applies to the scapular-side implant, which acts as the slide partner to the artificial humerus head. The artificial joint also includes a bearing structure, usually made of polyethylene, with a bearing surface opposite the humerus head.

Modular artificial joint systems generally comprise a carrier shell for a bearing structure incorporating the bearing surface. The carrier shell is generally made of metal, in particular titanium or titanium alloy, and can include anchoring means in the form of "pegs" or "keels", with which to anchor the shell in the bone. Alternatively, the carrier shell can also be anchored in the bone by means of screws, adjusting screws or expandable pegs. Even when cement is used, anchoring means can be provided on the back surface of the implant to ensure better retention in the cement. Glenoid prostheses known in the art are further described, for example, in EP 0 903 127 A2, EP 1 013 246 A1 and EP 1 136 046 A2, the entirety of each of which is hereby incorporated by reference.

However, conventional artificial joint prostheses have a back surface that is medially convex in order to permit the implant to be inserted only in a direction that is substantially perpendicular to the shoulder blade, i.e. scapula. This applies in particular when anchoring means, in particular anchoring pegs, are also disposed on the medial side, wherein the anchoring means extend perpendicular thereto. As a result, additional processing (e.g., drilling, milling) is required in a direction parallel to the normal line of the original glenoid, for which a generous amount of space is a prerequisite. Such work can be done only by experienced operators.

Furthermore, with conventional implants there is a risk of the loosening of the glenoid prosthesis. If there is a mismatch between the radius of the ball (humerus-head replacement) and that of the socket (glenoid) due to the actual anatomy of the shoulder, such that the radius of the ball is smaller than the radius of the socket, a translational movement in the caudal-cranial direction becomes possible. This movement is limited by the edge of the socket (glenoid). When the ball pushes against the edge of the socket, a force is exerted on the socket that causes leverage out of the socket. This phenomenon is commonly known in the literature as the "rocking-horse" effect, and appears in both "keeled" and "pegged" implants.

Document US 4,964,865 discloses a glenoid prosthesis having a lateral surface for articulating with a humeral head and a flat medial surface which engages a flat resected surface on the scapula head. In one embodiment, an integral plastic prosthesis has a pair of pegs extending from a flat medial surface which are positioned in a pair of holes drilled in the bone. The mating flat surfaces and pegs and holes are bonded with cement. The flat mating surfaces resist rocking under applied superior-inferior loading and resection is simplified by providing a flat resected surface and holes drilled substantially perpendicular thereto.

Document FR 2 704 747 discloses a support intended to receive a glenoid element cooperating with a humeral head. This support is fixed in the glenoid cavity of the shoulder by means of diametrically arranged divergent screws in combination with a cylindrical and hollow central stud engaged in the neck of the glenoid cavity. In particular, the support comprises, at its peripheral edge, a transverse tab formed as a projection from its internal face in order to bear on the external part of the glenoid cavity.

Document US 5,782,924 discloses an artificial joint prosthesis comprising at least one leg projecting from the surface of the prosthesis. The leg has a major axis extending substantially parallel the surface and being tapered so as to snugly fit within a substantially corresponding configured channel formed in the bone adjacent its shaped surface.

### SUMMARY OF THE INVENTION

The present invention provides a glenoid prosthesis in accordance with independent claim 1. Preferred embodiments of the invention are reflected in the dependent claims. The claimed invention can be better understood in view of the embodiments described hereinafter. In general, the described embodiments describe preferred embodiments of the invention. The attentive reader will note, however, that some aspects of the described embodiments extend beyond the scope of the claims. To the respect that the described embodiments indeed extend beyond the scope of the claims, the described embodiments are to be considered supplementary background information and do not constitute definitions of the invention *per se.* This also holds for the subsequent "Brief Description of the Drawings" as well as the "Detailed Description of the Preferred Embodiments."

It is thus one objective of the present disclosure to provide a glenoid prosthesis that is easier to handle than conventional prostheses, and with which a processing parallel to the normal line of the joint surface (i.e., perpendicular to the joint surface) is replaced by a processing and also implantation in the anterior/posterior (A-P) direction. These advantages are intended to apply to both a cement-free and a cemented version of the prosthesis.

In accordance with one embodiment, a glenoid prosthesis is provided comprising a bearing having a lateral side that defines a concave bearing surface. The prosthesis also comprises a carrier shell having a medial side that comprises at least one anchoring element, wherein the medial side is generally flat.

The prosthesis also comprises a lateral bearing surface and at least one anchoring element on a medial side of the prosthesis, wherein the at least one anchoring element comprises a ridge extending in an anterior-posterior direction and an enlarged portion at a free end thereof. The prosthesis also comprises a carrier shell comprising the at least one anchoring element and a bearing structure comprising the lateral bearing surface, wherein the carrier shell and the bearing structure are fixed to each other.

A method for implanting a glenoid prosthesis is also disclosed. The method includes positioning a cutting guide on an anterior surface of the glenoid of a scapula, and removing bone from the scapula at the glenoid to a subchondral position to form a generally flat surface. The method also includes positioning a drill guide on the scapula, drilling at least one bore in the scapula in generally an anterior-posterior direction, and forming at least one slot in communication with the at least one bore, the slot extending to the generally flat surface. The method further comprises attaching the glenoid prosthesis to the scapula, wherein the glenoid prosthesis includes a generally flat medial surface and at least one anchoring element projecting from the medial side and extending in generally an anterior-posterior direction, wherein the glenoid prosthesis is attached to the scapula by inserting the at least one anchoring element into the bore and the slot in an anterior-to-posterior direction. In one embodiment, the glenoid prosthesis has two anchoring elements. In another example, a screw can be implanted in the anterior-to-posterior direction to facilitate fixation of the prosthesis. For example, the carrier shell can have a plate that, upon implantation, lies proximal the anterior glenoid neck, the plate defining a hole that allows a screw to be inserted therethrough. The hole can be threaded or unthreaded. In one example, the screw can extend between two anchoring elements. In another embodiment, the screw can be inserted in association with one anchoring element.

An alternative method for implanting a glenoid prosthesis comprises forming a generally flat surface on a lateral side of a patient's scapula. A glenoid prosthesis is attached to the patient's scapula, the glenoid prosthesis having a generally flat surface on a medial side thereof to engage the generally flat surface on the lateral side of the patient's scapula. The glenoid prosthesis also has at least one anchoring element attaching the prosthesis to the scapula, and a bearing surface on a lateral side thereof.

Another method for implanting a glenoid prosthesis comprises forming at least one bore in generally an anterior-posterior direction in a patient's scapula, the bore communicating with a lateral side of the patient's scapula. A glenoid prosthesis is attached to the patient's scapula, the prosthesis having at least one anchoring element on a medial side thereof, wherein the prosthesis is attached by inserting the anchoring element through the at least one bore such that the medial side of the glenoid prosthesis engages the lateral side of the patient's scapula.

### BRIEF DESCRIPTION OF THE DRAWINGS

Embodiments of a glenoid prosthesis are described in greater detail below with reference to the attached drawings, wherein

Fig. 1 is a perspective side view of one embodiment of a glenoid prosthesis.

Fig. 2 is a perspective rear view of one embodiment of a carrier shell of the prosthesis according to Fig. 1.

Fig. 3 is a perspective side view of the carrier shell according to Fig. 1 and/or Fig. 2.

Fig. 4 is a perspective rear view of one embodiment of a bearing structure of the prosthesis according to Fig. 1.

Fig. 5 is a schematic front view of a conventional total prosthesis for a shoulder joint.

Fig. 6 is a schematic front view of a total prosthesis, including a glenoid prosthesis.

### DETAILED DESCRIPTION OF THE PREFERRED EMBODIMENTS

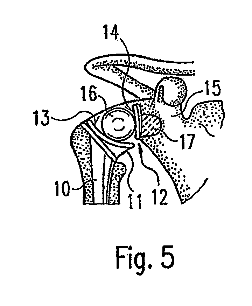

For better understanding of the context of the glenoid prosthesis in accordance with the disclosure, a shoulder-joint total prosthesis according to the state of the art will be described with reference to Fig. 5, which shows a humerus nail 10 with a bearing structure 11. The bearing structure 11 defines a concave bearing surface 13 for a joint or humerus ball 16. On the scapula side, the joint ball 16 is supported against a glenoid prosthesis 12, which has a bearing surface 14 that likewise has a concave curvature. The glenoid prosthesis 12 is anchored in the scapula, i.e. in the shoulder blade 15, where said anchoring is reinforced by an anchoring peg 17.

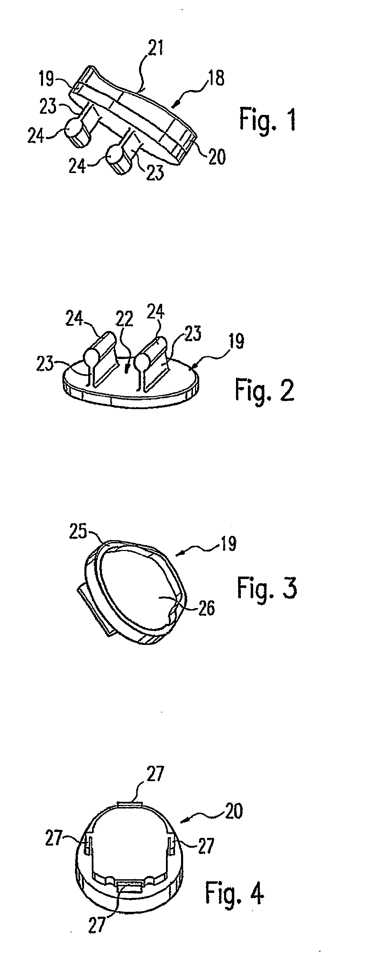

Embodiments are concerned with further development of the glenoid prosthesis 12, as discussed with reference to Figs. 1-4.

Figure 1 shows a glenoid prosthesis 18 comprising a carrier shell 19 preferably made of a human-tissue-compatible metal, such as titanium or a titanium alloy, and a bearing structure 20 preferably made of plastic, such as polyethylene, a ceramic material or metal. However, the carrier shell 19 can also be made of a plastic material, such as a plastic-fiber composite, that exhibits a similar strength as metal. The bearing structure 20 is positioned on the lateral side of the implant and defines a concave bearing surface 21. The concave bearing surface 21 can comprise at least two spherical surfaces differing from one another in radius. Alternatively the bearing surface can be part of an ellipsoid, hyperboloid or a paraboloid surface. Preferably, the medial (i.e., back) side 22 of the implant (see Figure 2), specifically of the carrier shell 19, is generally flat, and more preferably may be substantially or completely flat forming a planar area from which two ridges 23 spaced apart from one another project. Each of the ridges 23 extend in the anterior/posterior direction and has an expanded portion 24 at its free end. In the illustrated embodiment, the expanded portion 24 has a cylindrical shape with a round cross-section along a plane generally perpendicular to the anterior-posterior direction. In other embodiments, the cross-section of the expanded portion 24 can be rectangular, trapezoidal, or oval. The illustrated embodiment shows two ridges 23 extending from the medial side 22 of the carrier shell 19, each ridge 23 having an expanded portion 24 at a free end thereof. However, in another embodiment the carrier shell 19 can have one ridge 23 with an expanded portion 24 extending from the medial side 22 thereof. In still another embodiment, a screw can be implanted in an A-P direction to facilitate fixation of the prosthesis 18. For example, the carrier shell 19 can have a plate that, upon implantation, lies proximal the anterior glenoid neck, the plate defining a hole that allows the screw to be inserted therethrough. The screw can be threaded or unthreaded. In one embodiment, the screw can extend between two ridges 23. In another embodiment, the screw can be inserted in association with one ridge 23.

The embodiment illustrated in Figs. 1-4 is modular. Specifically, the prosthesis 18 comprises a separate carrier shell 19, as well as a bearing structure 20 that attaches thereto and has a bearing surface 21. The bearing structure 20 is preferably fastened to the carrier shell 19 in a force-fitting and/or form-fitting manner. Additionally, the bearing structure 20 and carrier shell 19 can be fastened together in-situ or prior to implantation.

In the illustrated embodiment, the carrier shell 19 and bearing structure 20 are fastened together by a catch means. In another embodiment, the carrier shell 19 and bearing structure 20 can form part of a monoblock or one-piece glenoid prosthesis. In the illustrated embodiment, the lateral side of the carrier shell 19 is trough-shaped, as shown in Fig. 3 and comprises a circumferential rim 25 bordering a generally flat floor 26. The inside of the circumferential rim 25 preferably has at least one undercut portion sized to receive and engage a complementary projection 27 on the associated side of the bearing structure 20. Accordingly, in the illustrated embodiment, the catch means includes the undercut portions of the carrier shell 19 and the projections 27 of the bearing structure 20.

The undercut portions can include recesses, slots, grooves, or other configurations suitable for lockably engaging the projections 27. In one embodiment, the undercut portion can extend along the circumference of the rim 25. In another embodiment, the undercut portions can be formed at discrete locations about the circumference of the rim 25.

In the illustrated embodiment, a total of four projections 27 are provided on the underside of the bearing structure 20, which correspond to complementary undercuts on the inside of the circumferential rim 25 of the carrier shell 19. However, any number of suitable projections 27 can be provided to fasten the carrier shell 19 and bearing structure 20 together.

As shown in Figure 4, the projections 27 are preferably equidistantly positioned around the periphery of the bearing structure 20. In another embodiment, the projections can be positioned at non-equidistant locations about the periphery of the bearing structure 20. However, any suitable mechanism for fastening the carrier shell and bearing structure can be used. As shown in Fig. 1, when assembled the circumferential surface of the bearing structure 20 is substantially flush with the outer circumference of the circumferential rim 25.

In the illustrated embodiment, the glenoid prosthesis 18 is modular in construction. In another embodiment, the glenoid prosthesis can also be formed as one piece, which can be made of plastic, ceramic or metal. Additionally, in one embodiment the carrier shell 19 and the bearing structure 20 are removably fastened to each other. In another embodiment, the bearing structure 20 is permanently attached to the carrier shell 19, so as to form a quasi-integral prosthesis.

Preferably, the glenoid prosthesis 18 shown in Figures 1-4 has an elongated oval shape, which can be formed using any suitable manner known in the art. However, the glenoid prosthesis 18 can have other shapes, such as circular.

In one embodiment, the prosthesis 18 comprises the use of cement. In such an embodiment, the medial side of the implant 18 corresponds to that of the medial side of the shell 19, and the lateral side of the implant corresponds to that of the lateral side of the bearing structure 20.

In one embodiment, where the bearing structure 20 is made of a ceramic or metallic material, the prosthesis 18 preferably includes an intermediate layer. The intermediate layer is preferably disposed between the carrier shell 19 and the bearing structure 20 and is made of a plastic, such as PE or PEEK.

The bores in the bone that correspond to the ridge expansions 24 are preferably formed with a drill guide, which can be positioned on an anterior surface of the scapula 15 with the bores formed by drilling in an anterior-posterior direction. In one embodiment, the drill guide can be fastened to the scapula with a k-wire. However, other suitable mechanisms can be used to fasten the drilling guide.

A surgical procedure for implanting the glenoid prosthesis 18 comprises resecting bone from the scapula. Preferably, bone is resected to a subchondral position so as to provide a generally planar surface to correspond to the planar surface of the medial side 22 of the carrier shell 19. The drill guide described above may be used to produce the planar surface, or alternatively, a separate cutting guide may be used to resect bone, which may be attached to an anterior surface of the glenoid of the scapula with a K-wire. After resection of bone, a keyhole or drill guide may be positioned on an anterior surface of the scapula proximal the location of the glenoid, preferably fixed to the scapula with a K-wire. In one preferred embodiment, the drill guide has a generally L-shaped body and preferably includes at least one elongated bore formed on an anterior member of the body, more preferably two elongated bores, corresponding in size to the enlarged expansions 24 of the carrier shell 19. Adjacent each of the bores, on a lateral member of the body that extends generally perpendicular to the anterior member, slots may be provided in the drill guide corresponding in size to the ridges 23 of the carrier shell 19. The drill guide is preferably positioned so that the anterior member is adjacent an anterior surface of the scapula 15 and so the lateral member is adjacent the planar surface on the glenoid. Bores are then preferably drilled into the scapula in an anterior-to-posterior direction through the bores in the drill guide. Additionally, slots are preferably formed on the scapula through the slots in the drill guide, so that each bore drilled in the scapula communicates with a corresponding slot. In one embodiment, the slots are formed in an anterior-to-posterior direction. In another embodiment, the slots are formed in a lateral-to-medial direction on the planar surface of the glenoid through slots in the lateral member. In yet another embodiment, the slots are formed in both an anterior-to-posterior direction and a medial-to-lateral direction. These slots may be formed to extend from the bores to the planar surface on the lateral side of the scapula. The bores thus obtained may be connected by way of saw-cuts to the flattened bearing surface for the glenoid prosthesis 18.

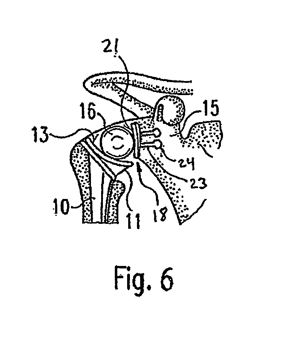

Said drill guide can also be simultaneously used as a cutting guide for preparation of the planar contact surface on the scapula for contact with the glenoid implant 18. The prosthesis 18 can then be implanted in an anterior-posterior direction, so that the ridge expansions 24 extend into the bores drilled in the scapula, and so the anchoring ridges extend through the slots connected to the bores, as shown in Figure 6 (showing a single ridge embodiment).

The glenoid prosthesis 18 shown in Figs. 1-4 is preferably anchored in bone in a form-fitting manner, so as to inhibit the leveraging out of the implant. Advantageously, the cylindrically-shaped ridge expansions 24 inhibit the "rocking-horse" effect discussed above. This applies to both the cemented and the cement-free versions of the implant 18.

In one preferred embodiment, the overall thickness of the prosthesis 18, i.e. of carrier shell 19 plus bearing structure 20, is between about 3.0 mm and 10.0 mm. In another embodiment, the overall thickness of the prosthesis 18 is about 5.0 mm to 7.5 mm. However, the prosthesis 18 can have other suitable thicknesses.

In one embodiment, the bearing structure 20 can be fastened to the carrier shell 19 using screws. In another embodiment, a dovetailed fastener arrangement can be used, so that the bearing structure 20 can be inserted into the carrier shell 19 from one side, whereupon the dovetailed fastener can engage the bearing structure 20 and carrier shell 19.

In one embodiment, the medial side of the carrier shell 19 is preferably roughened in any suitable manner known in the art in order to facilitate bone growth onto it. In one preferred embodiment, a grit-blasted, titanium plasma sprayed, Hydroxylapatite (HA) coating or other on/in-growth coatings used in joint replacement is applied to the carrier shell 19 to facilitate bone growth onto it. The medial side and/or the anchoring elements 24 also preferably comprise recesses (not shown) to enhance the process of bone growth into the prosthesis 18.

Advantageously, because the medial side of the glenoid prosthesis 18 is generally flat, more preferably substantially or completely flat and extending in the form of a plane, the processing of the scapular surface with a complementary shape is made correspondingly simple. Furthermore, the configuration of the medial side allows implantation in the anterior-posterior (A-P) direction, especially when the anchoring elements on the medial side extend in the A-P direction. In the case of cement-free implantation, of course, the scapular side should also be correspondingly prepared. This is done by means of a bone-processing tool that preferably operates in the anterior/posterior direction. As a result, processing of the scapular side parallel to the normal line of the original glenoid can be avoided, and considerably less processing space is needed than with conventional prostheses, thus reducing the corresponding stress imposed on the patient.

In another embodiment, the prosthesis 18, and in particular the carrier shell 19, can also comprise means for fixation of ball heads. For example, a ball head or hemisphere may be attached to or integrally formed with the lateral side of the of the carrier shell to form a reverse shoulder prosthesis (e.g., a glenosphere). In particular, the bearing structure 20 as described above may have a spherical or hemispherical shape to form the reverse shoulder prosthesis. This ball head replaces the ball 16 shown in FIGURE 6, and acts as a bearing surface against a humerus side bearing surface. Screws, pegs, adhesive, force-fitting or other suitable means may be used to attach the ball head to the carrier shell.

Of course, the foregoing description is that of certain features, aspects and advantages, to which various changes and modifications can be made without departing from the spirit and scope of the present disclosure. Moreover, the glenoid prosthesis need not feature all of the objects, advantages, features, and aspects discussed above. Thus, for example, those skilled in the art will recognize that the arrangement can be embodied or carried out in a manner that achieves or optimizes one advantage or a group of advantages as taught herein without necessarily achieving other objects or advantages as may be taught or suggested herein. In addition, while a number variations have been shown and described in detail, other modifications and methods of use, which are within the scope of this disclosure, will be readily apparent to those of skill in the art based upon this disclosure. It is contemplated that various combinations or sub-combinations of these specific features and aspects of embodiments may be made and still fall within the scope of the disclosure. According, it should be understood that various features and aspects of the disclosed embodiments can be combined with or substituted for one another in order to form varying modes of the discussed glenoid prosthesis.

### List of reference numerals

- 10: Humerus nail
- 11: Bearing structure
- 12: Glenoid prosthesis
- 13: Bearing surface
- 14: Bearing surface
- 15: Scapula (shoulder blade)
- 16: Joint ball
- 17: Anchoring peg
- 18: Glenoid prosthesis
- 19: Carrier shell
- 20: Bearing structure
- 21: Bearing surface
- 22: Medial side
- 23: Anchoring ridge
- 24: Ridge expansion
- 25: Circumferential rim
- 26: Floor
- 27: Catch projection

## Claims

1. A glenoid prosthesis (18)
having a lateral side that defines a concave bearing surface (21) and
a medial side (22) that comprises at least one anchoring element (23, 24) attached thereto, wherein the medial side (22) is flat,
wherein the at least one anchoring element (23, 24) comprises a ridge (23) extending in an anterior/posterior, A-P, direction, so that in cement-free implantation the prosthesis (18) can be inserted in the A-P direction, the ridge (23) having a free end extending in the anterior/posterior direction,
**characterised in that** the glenoid prostesis comprises a bearing structure (20) and a carrier shell (19),
wherein the concave bearing surface (21) has a shape selected from the group consisting of an ellipsoid, a hyperboloid and a paraboloid surface.

2. A prosthesis (18) according to Claim 1, wherein the at least one anchoring element (23, 24) comprises two ridges (23) spaced apart from one another.

3. A prosthesis (18) according to Claim 1, wherein the free ends of the anchoring ridges (23) have ridge expansion portions (24).

4. A prosthesis (18) according to Claim 3, wherein the ridge expansion portions (24) have a cross-sectional shape selected from the group consisting of rectangular, trapezoidal, oval and cylindrical.

5. A prosthesis (18) according to any one of Claims 1-3, wherein the prosthesis, (18) is of modular construction and said bearing structure (20) comprises said bearing surface (21), the carrier shell (19) and bearing structure (20) being configured to fasten to each other.

6. A prosthesis (18) according to any one of Claims 1-4, wherein an intermediate layer of plastic is disposed between carrier shell (19) and bearing structure (20).

7. A prosthesis (18) according to Claim 6, wherein the intermediate layer is made of PE or PEEK.

8. A prosthesis (18) according to any one of Claims 1-7, wherein at least one of the bearing surface (21) and the bearing structure (20) comprises an oval or circular shape in plan view.

9. A prosthesis (18) according to any one of Claims 1-8, wherein the concave bearing surface (21) comprises at least two spherical surfaces differing in radius.

10. A prosthesis (18) according to any one of Claims 1-9, wherein the bearing structure (20) comprising the bearing surface (21) is made of a material selected from the group consisting of plastic, polyethylene, ceramic, metal, and a composite thereof, the bearing structure (20) fastened to the carrier shell (19) either in situ or prior to implantation.

11. A prosthesis (18) according to any one of Claims 5-10, wherein the bearing structure (20) is fastened to the carrier shell (19) using a catch means in a manner selected from the group consisting of a force-fitting and a form-fitting manner.

12. A prosthesis (18) according to any of Claims 1-11, wherein the carrier shell (19) is made of a metal selected from the group consisting of titanium, titanium alloy, plastic, and a plastic-fiber composite.

13. A prosthesis (18) according to any of Claims 1-12, wherein the medial side (22) is roughened.

14. A prosthesis (18) according to any of Claims 1-13, wherein the medial side (22) is coated to facilitate bone growth onto the carrier shell (19).

15. A prosthesis (18) according to any of Claims 1-14, wherein at least one of the medial side (22) and the at least one anchoring element (23, 24) comprises recesses thereon to improve a connection between the prosthesis (18) and the receiving bone via bone growth in said recesses.

16. A prosthesis (18) according to any of Claims 1-15, wherein the carrier shell (19) comprises means for the fixation of spherical heads thereon.

## Patentansprüche

1. Eine Glenoidprothese (18), die eine laterale Seite, die eine konkave Auflagefläche (21) definiert, und eine mediale Seite (22), die mindestens ein daran befestigtes Verankerungselement (23, 24) beinhaltet, aufweist, wobei die mediale Seite (22) flach ist,
wobei das mindestens eine Verankerungselement (23, 24) eine Erhöhung (23) beinhaltet, die sich in eine anteriore/posteriore A-P-Richtung erstreckt, so dass bei der zementfreien Implantation die Prothese (18) in der A-P-Richtung eingeführt werden kann, wobei die Erhöhung (23) ein freies Ende aufweist, das sich in die anteriore/posteriore Richtung erstreckt,
**dadurch gekennzeichnet, dass** die Glenoidprothese eine Auflagestruktur (20) und eine Trägerschale (19) beinhaltet
wobei die konkave Auflagefläche (21) eine Form aufweist, die aus der Gruppe, bestehend aus einer ellipsoiden, einer hyperboloiden und einer paraboloiden Fläche, ausgewählt ist.

2. Prothese (18) gemäß Anspruch 1, wobei das mindestens eine Verankerungselement (23, 24) zwei Erhöhungen (23) beinhaltet, die mit Abstand voneinander angeordnet sind.

3. Prothese (18) gemäß Anspruch 1, wobei die freien Enden der Verankerungserhöhungen (23) Erhöhungsausdehnungsabschnitte (24) aufweisen.

4. Prothese (18) gemäß Anspruch 3, wobei die Erhöhungsausdehnungsabschnitte (24) eine Querschnittsform aufweisen, die aus der Gruppe, bestehend aus rechteckig, trapezoid, oval und zylindrisch, ausgewählt ist.

5. Prothese (18) gemäß einem der Ansprüche 1-3, wobei die Prothese (18) von modularer Konstruktion ist und die Auflagestruktur (20) die Auflagefläche (21) beinhaltet, wobei die Trägerschale (19) und die Auflagestruktur (20) konfiguriert sind, um aneinander festgemacht zu werden.

6. Prothese (18) gemäß einem der Ansprüche 1-4, wobei eine Zwischenschicht aus Kunststoff zwischen der Trägerschale (19) und der Auflagestruktur (20) angeordnet ist.

7. Prothese (18) gemäß Anspruch 6, wobei die Zwischenschicht aus PE oder PEEK gefertigt ist.

8. Prothese (18) gemäß einem der Ansprüche 1-7, wobei mindestens eine der Auflagefläche (21) und der Auflagestruktur (20) in der Draufsicht eine ovale oder kreisförmige Form beinhaltet.

9. Prothese (18) gemäß einem der Ansprüche 1-8, wobei die konkave Auflagefläche (21) mindestens zwei sich im Radius unterscheidende Kugelflächen beinhaltet.

10. Prothese (18) gemäß einem der Ansprüche 1-9, wobei die Auflagestruktur (20), die die Auflagefläche (21) beinhaltet, aus einem Material gefertigt ist, das aus der Gruppe, bestehend aus Kunststoff, Polyethylen, Keramik, Metall und einer Zusammensetzung davon, ausgewählt ist, wobei die Auflagestruktur (20) entweder in situ oder vor der Implantation an der Trägerschale (19) festgemacht wird.

11. Prothese (18) gemäß einem der Ansprüche 5-10, wobei die Auflagestruktur (20) unter Verwendung eines Sperrmittels auf eine Weise, ausgewählt aus der Gruppe, bestehend aus einer kraftgerechten und einer körpergerechten Weise, an der Trägerschale (19) festgemacht ist.

12. Prothese (18) gemäß einem der Ansprüche 1-11, wobei die Trägerschale (19) aus einem Metall, ausgewählt aus der Gruppe, bestehend aus Titan, Titanlegierung, Kunststoff und einer Kuntstoff-Faser-Zusammensetzung, gefertigt ist.

13. Prothese (18) gemäß einem der Ansprüche 1-12, wobei die mediale Seite (22) geraut ist.

14. Prothese (18) gemäß einem der Ansprüche 1-13, wobei die mediale Seite (22) beschichtet ist, um das Knochenwachstum auf die Trägerschale (19) zu erleichtern.

15. Prothese (18) gemäß einem der Ansprüche 1-14, wobei mindestens eines von der medialen Seite (22) und dem mindestens einen Verankerungselement (23, 24) Aussparungen darauf beinhaltet, um eine Verbindung zwischen der Prothese (18) und dem empfangenden Knochen über Knochenwachstum in den Aussparungen zu verbessern.

16. Prothese (18) gemäß einem der Ansprüche 1-15, wobei die Trägerschale (19) ein Mittel zur Fixierung der Kugelköpfe darauf beinhaltet.

## Revendications

1. Une prothèse glénoïde (18) présentant un côté latéral qui définit une surface d'appui concave (21) et un côté médial (22) qui comprend au moins un élément d'ancrage (23, 24) attaché à celui-ci, le côté médial (22) étant plat,
dans laquelle l'au moins un élément d'ancrage (23, 24) comprend une arête (23) s'étendant dans une direction antérieure / postérieure, A-P, si bien que, dans une implantation sans ciment, la prothèse (18) peut être insérée dans la direction A-P, l'arête (23) présentant une extrémité libre s'étendant dans la direction antérieure / postérieure,
**caractérisée en ce que** la prothèse glénoïde comprend une structure d'appui (20) et une coque porteuse (19),
la surface d'appui concave (21) ayant une conformation sélectionnée dans le groupe constitué par une surface ellipsoïde, une surface hyperboloïde et une surface paraboloïde.

2. Une prothèse (18) selon la revendication 1, dans laquelle l'au moins un élément d'ancrage (23, 24) comprend deux arêtes (23) espacées l'une de l'autre.

3. Une prothèse (18) selon la revendication 1, dans laquelle les extrémités libres des arêtes d'ancrage (23) présentent des portions d'expansion d'arête (24).

4. Une prothèse (18) selon la revendication 3, dans laquelle les portions d'expansion d'arête (24) ont une conformation en coupe transversale sélectionnée dans le groupe constitué par des conformations rectangulaire, trapézoïdale, ovale et cylindrique.

5. Une prothèse (18) selon l'une quelconque des revendications 1 à 3, dans laquelle la prothèse (18) est de construction modulaire et ladite structure d'appui (20) comprend ladite surface d'appui (21), la coque porteuse (19) et la structure d'appui (20) étant configurées pour s'assujettir l'une à l'autre.

6. Une prothèse (18) selon l'une quelconque des revendications 1 à 4, dans laquelle une couche intermédiaire de plastique est disposée entre la coque porteuse (19) et la structure d'appui (20).

7. Une prothèse (18) selon la revendication 6, dans laquelle la couche intermédiaire est réalisée en PE ou en PEEK.

8. Une prothèse (18) selon l'une quelconque des revendications 1 à 7, dans laquelle au moins soit la surface d'appui (21), soit la structure d'appui (20) comprend une conformation ovale ou circulaire en vue en plan.

9. Une prothèse (18) selon l'une quelconque des revendications 1 à 8, dans laquelle la surface d'appui concave (21) comprend au moins deux surfaces sphériques dont les rayons diffèrent.

10. Une prothèse (18) selon l'une quelconque des revendications 1 à 9, dans laquelle la structure d'appui (20) comprenant la surface d'appui (21) est réalisée en une matière sélectionnée dans le groupe constitué par du plastique, du polyéthylène, de la céramique, du métal, et un composite de ceux-ci, la structure d'appui (20) étant assujettie à la coque porteuse (19) soit in situ, soit préalablement à l'implantation.

11. Une prothèse (18) selon l'une quelconque des revendications 5 à 10, dans laquelle la structure d'appui (20) est assujettie à la coque porteuse (19) en utilisant un moyen de prise d'une manière sélectionnée dans le groupe constitué par une manière d'ajustement à force et une manière d'ajustement moulant.

12. Une prothèse (18) selon n'importe lesquelles des revendications 1 à 11, dans laquelle la coque porteuse (19) est réalisée en un métal sélectionné dans le groupe constitué par le titane, l'alliage de titane, en plastique, et en un composite à fibres de plastique.

13. Une prothèse (18) selon n'importe lesquelles des revendications 1 à 12, dans laquelle le côté médial (22) est rugosifié.

14. Une prothèse (18) selon n'importe lesquelles des revendications 1 à 13, dans laquelle le côté médial (22) est enduit afin de faciliter une croissance osseuse sur la coque porteuse (19).

15. Une prothèse (18) selon n'importe lesquelles des revendications 1 à 14, dans laquelle au moins soit le côté médial (22), soit l'au moins un élément d'ancrage (23, 24) comprend des renfoncements sur celui-ci afin d'améliorer un raccordement entre la prothèse (18) et l'os receveur via une croissance osseuse dans lesdits renfoncements.

16. Une prothèse (18) selon n'importe lesquelles des revendications 1 à 15, dans laquelle la coque porteuse (19) comprend un moyen destiné à la fixation de têtes sphériques sur celle-ci.
